# EUROPÄISCHE PATENTSCHRIFT

(11) **EP 1 935 394 B1**
(45) Veröffentlichungstag und Bekanntmachung des Hinweises auf die Patenterteilung: **03.11.2010**
(21) Anmeldenummer: 07033510.4
(22) Anmeldetag: 27.08.2007
(51) Int. Cl.: A61K 8/06, A61K 8/34, A61K 8/37, A61K 8/46, A61K 8/49, A61Q 17/04, A61Q 19/00

(54) **Leichte Lotion**
Light lotion
Lotion légère

(30) Priorität: 22.12.2006 DE 102006061689
(43) Veröffentlichungstag der Anmeldung: 25.06.2008
(73) Patentinhaber: Beiersdorf Aktiengesellschaft, 20253 Hamburg (DE)
(72) Erfinder: Lerg, Heike, 22303 Hamburg (DE); Mundt, Claudia, Dr., 8073 Zürich (CH); Steikert, Claudia, 25469 Halstenbek (DE); Sugár, Martin, Dr., 20253 Hamburg (DE); Boenisch, Alexandra, 20257 Hamburg (DE)

(56) Entgegenhaltungen:
- EP-A- 1 077 059
- EP-A- 1 380 288
- EP-A- 1 557 160
- EP-A- 1 764 083
- DE-A1- 4 342 719
- DE-A1- 10 162 697
- DE-A1- 19 839 402
- DE-A1-102004 027 476

## Beschreibung

Die vorliegende Erfindung betrifft eine kosmetische Lotion.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut.

Hautpflegeprodukte (sowie einige Hautreinigungsprodukte) bestehen in der Regel aus Emulsionen. Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Dünnflüssige Emulsionen, d.h. Emulsionen mit einer Viskosität von 500 bis 2500 mPas, besonders bevorzugt mit einer Viskosität von 1200 bis 2000 mPas bezeichnet man im allgemeinen als "Lotionen". Lotionen sind bei den Anwendern besonders beliebt, da sie sich leicht auf der Haut verteilen lassen und schnell in die Haut einziehen. Darüber hinaus sind sie im Regelfall sprühbar. Die Viskositätsmessung wird mit dem Viskotester VT 02 der Firma Haake bei 25°C +/- 1°C., Spindel 1 durchgeführt. Die Ablesung erfolgt nach 30 Sekunden.

Nachteilig am Stande der Technik ist jedoch der Umstand, dass herkömmliche Lotionen nach dem Auftragen beim Schwitzen ein klebriges Hautgefühl erzeugen. Beim starken Schwitzen (beispielsweise, wenn es sich bei der Lotion um ein Sonnenschutzmittel handelt, dass im Hochsommer angewendet wird) kommt es darüber hinaus zu einer weißlichen Schlierenbildung auf der Haut. Die gleichmäßige Verteilung des Sonnenschutzmittels wird damit aufgehoben; die einzelnen Hautpartien sind ungleichmäßig vor der Sonnenstrahlung geschützt.

Es war daher die Aufgabe der vorliegenden Erfindung, eine Lotion zu entwickeln, welche die Nachteile des Standes der Technik nicht mehr aufweist.

Überraschend gelöst wird die Aufgabe durch eine kosmetische Emulsion enthaltend eine Kombination aus
a) 0,5 bis 3 Gew.-% Glycerylstearat SE
b) 1 bis 5 Gew.-% Cetearylalkohol,
c) 0,01 bis 0,5 Gew.-% PEG-40 Rizinusöl,
d) 0,05 bis 0,25 Gew.-% Natriumcetearylsulfat und
e) 0,5 bis 3 Gew.-% Myristylmyristat, jeweils bezogen auf das Gesamtgewicht der Zubereitung.

Die erfindungsgemäßen Zubereitungen ziehen überraschend schnell in die Haut ein.

Darüber hinaus war es überraschend, dass die Zubereitungen besonders wenig "weißeln". Unter dem Effekt des "Weißelns" wird dabei erfindungsgemäß ein Phänomen verstanden, welches beispielsweise in "Sensory Evaluation Techniques, 3rd Edition, Meilgaard, Civille, Carr, CRC Press Boca Raton, London, New York, Washigton, D.C., Seite 184-185 ("Terms used to describe skinfeel of lotions and creams)" beschrieben ist und sich mit einem sensorischen Panel-Versuch einfach nachweisen lässt. Dabei wird 0,1 ml des Produktes auf einer definierten Fläche auf der Oberseite der Unterarme verteilt. Während dieses Vorgangs wird beurteilt, in welchem Maße das Produkt weiß aufliegt und wie es dauert, bis es verschwindet. Die Abstufung erfolgt auf einer 10-stufigen Skala, wobei 0=weißelt nicht / und 10= weißelt stark bedeutet.

Zwar kennt der Stand der Technik die DE 19839402, EP 1557160, DE 102004027476, EP 1380288, EP 1077059, DE 10162697, DE 4342719 und EP 1764083, doch konnten diese Schriften nicht den Weg zur vorliegenden Erfindung weisen.

Bei Glycerylstearat SE (INCI Glyceryl Stearate SE) handelt es sich um Verbindungen von Glycerin mit Stearinsäure (Glycerylmonostearat) und einem Anteil von Natrium- und/oder Kaliumstearat, kommerzialisiert von der Firma Goldschmidt unter dem Namen TEGIN (CTFA Name: Glyceryl Stearat SE, CAS-Nr 11099-07-3 (Glycerinmonodistearat/palmitat) und 593-29-3 (Kaliumstearat/palmitat)

Es ist erfindungsgemäß vorteilhaft, wenn die erfindungsgemäße Zubereitung als weitere Bestandteile einen oder mehrere UV-Lichtschutzfilter enthält.

Erfindungsgemäß vorteilhafte Ausführungsformen sind dabei dadurch gekennzeichnet, dass die Zubereitung einen oder mehrere UV-Lichtschutzfilter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bornylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazol-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester, 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin (INCI :Bis-Ethylhexyloxyphenol Methoxyphenyl Triazin); Dioctylbutylamidotriazon (INCI: Diethylhexyl-Butamidotriazone); 2,4-bis-[5-1 (dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoäsäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Octyl 5-N,N-diethylamino-2-phenylsulphonyl-2,4-pentadienoat, Merocyanine wie sie in der WO 2006/125676 und WO 2004/006878 beschrieben sind; Benzoylzimtsäurenitrile, Titandioxid; Zinkoxid in einer Konzentration von 0,01 bis 40 Gewichts-% bezogen auf das Gesamtgewicht der Zubereitung.

Es ist erfindungsgemäß bevorzugt, wenn die Zubereitung dabei einen oder mehrere UV-Filter gewählt aus der Gruppe Titandioxid, 4,4',4"-(1,3,5-Triazin-2,4,6-triyltriimino)-tris-benzoësäuretris(2-ethylhexylester), 4-Methoxyzimtsäure(2-ethylhexyl)ester, 4-(tert.-Butyl)-4'-methoxydibenzoylmethan, 2-Phenylbenzimidazol-5-sulfonsäuresalze, 2,4-Bis-{[4-(2-ethyl-hexyloxy)-2-hydroxy]-phenyl}-6-(4-methoxyphenyl)-1,3,5-triazin enthält, wobei eine Kombination aus allen diesen Filtern erfindungsgemäß besonders bevorzugt ist.

Es ist erfindungsgemäß vorteilhaft, wenn die erfindungsgemäße Zubereitung frei ist von p-Methylbenzylidencampher.

Die Pigmente (Titandioxid, Zinkoxid) können vorteilhaft im Sinne der vorliegenden Erfindung auch in Form kommerziell erhältlicher öliger oder wäßriger Vordispersionen zur Anwendung kommen. Diesen Vordispersionen können vorteilhaft Dispergierhilfsmittel und/oder Solubilisationsvermittler zugesetzt sein.

Die Pigmente (Titandioxid, Zinkoxid) können erfindungsgemäß vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), Bariumsulfat (BaSO₄) oder Eisenoxid (Fe₂O₃). Diese anorganischen Oberflächenbeschichtungen können allein, in Kombination und/oder in Kombination mit organischen Beschichtungsmaterialien vorkommen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Erfindungsgemäß bevorzugte Ausführungsformen der vorliegenden Erfindung sind dadurch gekennzeichnet, dass die Zubereitung als UV-Filter Titandioxid in einer Konzentration von 0,1 bis 5 Gew.-%, bezogen auf das Gesamtgewicht der Zubereitung enthält.

Es ist dabei erfindungsgemäß besonders bevorzugt, wenn die Zubereitung Titandioxid in einer Konzentration von 0 bis 5 Gew.-%, bezogen auf das Gesamtgewicht der Zubereitung enthält.

Erfindungsgemäß vorteilhafte Ausführungsformen der vorliegenden Erfindung sind dadurch gekennzeichnet, dass die erfindungsgemäße Zubereitung ein oder mehrere Verbindungen gewählt aus der Gruppe der Parabene, Phenoxyethanol, Ethylhexylglycerin, 2-Methylpropan-1,3-diol, Butylenglycol, Propylenglycol enthält, die in einer Gesamtkonzentration von 0,01 bis 10 Gewichts-%, bezogen auf das Gesamtgewicht der Zubereitung in dieser vorliegen können.

Erfindungsgemäß vorteilhafte Ausführungsformen der vorliegenden Erfindung sind dadurch gekennzeichnet, dass die Zubereitung bis zu 5 Gew.-% Glycerin, bezogen auf das Gesamtgewicht der Zubereitung, enthält. Dabei ist ein Gehalt an Glycerin von 0,1 bis zu 2 Gewichts-%, bezogen auf das Gesamtgewicht der Zubereitung erfindungsgemäß bevorzugt.

Erfindungsgemäß vorteilhafte Ausführungsformen der vorliegenden Erfindung sind dadurch gekennzeichnet, dass die Zubereitung bis zu 15 Gew.-% Ethanol, bezogen auf das Gesamtgewicht der Zubereitung, enthält. Dabei ist es erfindungsgemäß besonders bevorzugt, wenn der Gehalt an Ethanol 4 bis 10 Gew.-%, bezogen auf das Gesamtgewicht der Zubereitung beträgt.

Erfindungsgemäß vorteilhafte Ausführungsformen der vorliegenden Erfindung sind dadurch gekennzeichnet, dass die Zubereitung bis zu 5 Gew.-% Stärkederivate, bezogen auf das Gesamtgewicht der Zubereitung, enthält. Dabei ist ein Gehalt von 1 bis zu 4 Gew.-%, bezogen auf das Gesamtgewicht der Zubereitung erfindungsgemäß bevorzugt.

Es ist erfindungsgemäß vorteilhaft, wenn die Stärkederivate gewählt werden aus der Gruppe der Verbindungen Distärkephosphat, Natriumoctenylsuccinatstärke. Erfindungsgemäß besonders bevorzugt ist dabei eine Kombination aus beiden Stärkederivaten.

Erfindungsgemäß vorteilhaft enthält die erfindungsgemäße Zubereitung Xanthangummi. Dabei ist es erfindungsgemäß bevorzugt, wenn der Gehalt an Xanthangummi 0,1 bis 0,5 Gew.-%, bezogen auf das Gesamtgewicht der Zubereitung beträgt.

Erfindungsgemäß vorteilhaft enthält die erfindungsgemäße Zubereitung Vitamin E acetat. Diese Verbindung wird erfindungsgemäß bevorzugt in einer Konzentration von 0,01 bis 1 Gew.-%, bezogen auf das Gesamtgewicht der Zubereitung eingesetzt.

Es ist erfindungsgemäß vorteilhaft, wenn die erfindungsgemäße Zubereitung ein oder mehrere Ölkomponenten gewählt aus der Gruppe der Verbindungen C₁₂₋₁₅-Alkylbenzoat, Capryl-/Caprinsäure Triglycerid, Dicaprylcarbonat.enthält.

Es hat sich dabei als erfindungsgemäß besonders bevorzugt herausgestellt, diese Ölkomponenten in den folgenden Einsatzkonzentrationen (bezogen auf das Gesamtgewicht der Zubereitung) einzusetzen:
C₁₂₋₁₅-Alkylbenzoat: 4 bis 8 Gew.-%,
Capryl-/Caprinsäure Triglycerid: 0,5 bis 5 Gew.-%.

Erfindungsgemäß vorteilhaft enthält die erfindungsgemäße Zubereitung als weitere Inhaltsstoffe eine oder mehrere Verbindungen gewählt aus der Gruppe der Verbindungen Folsäure, Phytoen, D-Biotin, Coenzym Q10, alpha-Glucosylrutin, Carnitin, Polydocanol, natürliche und/oder synthetische Isoflavonoide, insbesondere Genistein, Flavonoide, Carotinoide, Kreatin, Kreatinin, Taurin, Ascorbinsäure + Derivate Sauerstoff, Dihydroxyaceton; 8-Hexadecen-1,16-dicarbonsäure, Glycerylglucose, lang-wie auch kurzkettige Hyaluronsäure (d.h. Hyaluronsäure mit einem mittleren Molekulargewicht von 1 Million bis 3 Million Dalton, wie auch 5000 Dalton - 1 Million Dalton) und/oder Licochalcon A. Derartige Inhaltsstoffe können jeweils in einer Einzelkonzentration von 0,01 bis 10 Gewichts-%, bezogen auf das Gesamtgewicht der Zubereitung, in dieser enthalten sein.

Es ist erfindungsgemäß vorteilhaft, wenn die erfindungsgemäße Zubereitung zusätzlich Harnstoff in einer Konzentration von 0,01 bis 50 Gewichts-%, bevorzugt in einer Konzentration von 0.1 bis 20 Gewichts% und besonders bevorzugt in einer Konzentration von 1 bis 15 Gewichts% bezogen auf das Gesamtgewicht der Zubereitung, enthält.

Ferner vorteilhaft können die erfindungsgemäßen Zubereitungen auch Repellentien zum Schutz vor Mücken, Zecken und Spinnen und dergleichen enthalten. Vorteilhaft sind z. B. N,N-Diethyl-3-methylbenzamid (Handelsbezeichnung: "DEET"), Dimethylphtalat (Handelsbezeichnung: Palatinol M, DMP), 1-Piperidincarbonsäure-2-(2-hydroxyethyl)-1-methylpropylester sowie insbesondere 3-(N-n-Butyl-N-acetyl-amino)-propionsäureethylester (unter dem Handelsnamen Insekt Repellent® 3535 bei der Fa. Merck erhältlich). Die Repellentien können sowohl einzeln als auch in Kombination eingesetzt werden.

Die Wasserphase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, 2-Methylpropan-1,3-diol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte, Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Carragenanen, Hydroxypropylmethylcellulose, besonders vorteilhaft aus der Gruppe der Polyacrylate, bevorzugt ein Polyacrylat aus der Gruppe der sogenannten Carbopole, beispielsweise Carbopole der Typen 980, 981, 1382, 2984, 5984, jeweils einzeln oder in Kombination. Weitere erfindungsgemäß vorteilhafte Verdicker sind solche mit der INCl-Bezeichnung Acrylates/C10-30 Alkyl Acrylate Crosspolymer (z. B. Pemulen TR 1, Pemulen TR 2, Carbopol 1328 von der Fa. NOVEON) sowie Aristoflex AVC (INCl: Ammonium Acryloyldimethyltaurate/VP Copolymer).

Erfindungsgemäß vorteilhaft enthält die erfindungsgemäße Zubereitung Filmbildner. Filmbildner im Sinne der vorliegenden Erfindung sind Stoffe unterschiedlicher Zusammensetzung, die durch die folgende Eigenschaft charakterisiert sind: Löst man einen Filmbildner in Wasser oder anderen geeigneten Lösungsmitteln und trägt die Lösung dann auf die Haut auf, so bildet er nach dem Verdunsten des Lösemittels einen Film aus, der im wesentlichen dazu dient, die Lichtfilter auf der Haut zu fixieren und so die Wasserfestigkeit des Produktes zu steigern.

Es ist insbesondere von Vorteil, die Filmbildner aus der Gruppe der Polymere auf Basis von Polyvinylpyrrolidon (PVP) 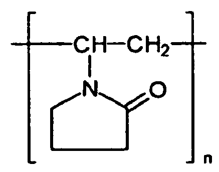 zu wählen. Besonders bevorzugt sind Copolymere des Polyvinylpyrrolidons, beispielsweise das PVP Hexadecen Copolymer und das PVP Eicosen Copolymer, welche unter den Handelsbezeichnungen Antaron V216 und Antaron V220 bei der GAF Chemicals Cooperation erhältlich sind.

Ebenfalls vorteilhaft sind weitere polymere Filmbildner, wie beispielsweise Natriumpolystryrensulfonat, welches unter der Handelsbezeichnung Flexan 130 bei der National Starch and Chemical Corp. erhältlich ist, und/oder Polyisobuten, erhältlich bei Rewo unter der Handelsbezeichnung Rewopal PIB1000. Weitere geeignete Polymere sind z.B. Polyacrylamide (Seppigel 305), Polyvinylalkohole, PVP, PVP / VA Copolymere, Polyglycole, Acrylat/Octylacralymid Copolymer (Dermacryl 79). Ebenfalls vorteilhaft ist die Verwendung von Hydriertem Rizinusöl Dimerdilinoleat (CAS 646054-62-8, INCI Hydrogenated Castor Oil Dimer Dilinoleate), das bei der Firma Kokyu Alcohol Kogyo unter dem Namen Risocast DA-H erworben werden kann oder aber auch PPG-3 Benzylethermyristat (CAS 403517-45-3), das unter dem Handelsnamen Crodamol STS bei der Firma Croda Chemicals erworben werden kann.

Besonders vorteilhafte Zubereitungen werden ferner erhalten, wenn als Zusatz- oder Wirkstoffe Antioxidantien eingesetzt werden. Erfindungsgemäß enthalten die Zubereitungen vorteilhaft eines oder mehrere Antioxidantien. Als günstige, aber dennoch fakultativ zu verwendende Antioxidantien können alle für kosmetische Anwendungen geeigneten oder gebräuchlichen Antioxidantien verwendet werden.

Besonders vorteilhaft im Sinne der vorliegenden Erfindung können wasserlösliche Antioxidantien eingesetzt werden, wie beispielsweise Vitamine, z. B. Ascorbinsäure und deren Derivate.

Die Menge der Antioxidantien (eine oder mehrere Verbindungen) in den Zubereitungen beträgt vorzugsweise 0,001 bis 30 Gew.-%, besonders bevorzugt 0,05 bis 20 Gew.-%, insbesondere 0,1 bis 10 Gew.-%, bezogen auf das Gesamtgewicht der Zubereitung.

Sofern Vitamin A bzw. Vitamin-A-Derivate, bzw. Carotine bzw. deren Derivate das oder die Antioxidantien darstellen, ist vorteilhaft, deren jeweilige Konzentrationen aus dem Bereich von 0,001 bis 10 Gew.-%, bezogen auf das Gesamtgewicht der Formulierung, zu wählen.

Es ist insbesondere vorteilhaft, wenn die kosmetischen Zubereitungen gemäß der vorliegenden Erfindung kosmetische Wirkstoffe enthalten, wobei bevorzugte Wirkstoffe Antioxidantien sind, welche die Haut vor oxidativer Beanspruchung schützen können.

Erfindungsgemäße Rezepturen, welche z. B. bekannte Antifaltenwirkstoffe wie Flavonglycoside (insbesondere α-Glycosylrutin), Coenzym Q10, Retinol und Ester, Vitamin E und/oder Derivate und dergleichen enthalten, eignen sich insbesondere vorteilhaft zum Schutz vor ästhetisch unattraktiven Hautveränderungen, wie sie z. B. bei der Hautalterung auftreten (wie beispielsweise Trockenheit, Rauhigkeit und Ausbildung von Trockenheitsfältchen, Juckreiz, verminderte Rückfettung (z. B. nach dem Waschen), sichtbare Gefäßerweiterungen (Teleangiektasien, Cuperosis), Schlaffheit und Ausbildung von Falten und Fältchen, lokale Hyper-, Hypo- und Fehlpigmentierungen (z. B. Altersflecken), vergrößerte Anfälligkeit gegenüber mechanischem Stress (z. B. Rissigkeit) und dergleichen) und ermüdete Haut. Weiterhin vorteilhaft eignen sie sich gegen das Erscheinungsbild der trockenen bzw. rauhen Haut.

Die kosmetischen Zubereitungen gemäß der Erfindung können kosmetische Hilfsstoffe enthalten, wie sie üblicherweise in solchen Zubereitungen verwendet werden, z. B. Konservierungsmittel, Konservierungshelfer, Komplexbildner, Bakterizide, Parfüme, Substanzen zum Verhindern oder Steigern des Schäumens, Farbstoffe, Pigmente, die eine färbende Wirkung haben, Verdickungsmittel, anfeuchtende und/oder feuchhaltende Substanzen, Füllstoffe, die das Hautgefühl verbessern, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösungsmittel oder Silikonderivate.

Erfindungsgemäß ist insbesondere die Verwendung der erfindungsgemäßen Zubereitung als Sonnenschutzmittel.

### Vergleichsversuche

### Testdesign:

- Selbstständiges eincremen einer Körperhälfte mit jeweils einem Produkt.
   o Muster 1 - erfindungsgemäß
   o Muster 2 - nicht erfindungsgemäß
- 15 min Produkte einziehen lassen.
- Saunaaufenthalt bis Proband (n=6) deutlich schwitzt (bis zu 30 Minuten)
- Visuelle Beurteilung des Schwitzens (Fotodokumentation) und Befragung der Probanden nach persönlichem Empfinden
-

### Rezeptur

### Muster 1:

| | |
|---|---|
| Glyceryl Stearat SE | 1 |
| Cetearyl Alkohol | 1,9 |
| PEG-40 Castoröl | 0,375 |
| Natrium Cetearyl Sulfat | 0,19 |
| Cetylalkohol | 1,5 |
| Myristylmyristat | 1,0 |
| Xanthan Gum | 0,4 |
| C₁₂₋₁₅ Alkyl Benzoat | 5,5 |
| Distarch Phosphat | 3 |
| Natriumstärke Octenylsuccinat | 1,5 |
| Glycerin | 0,75 |
| Alkohol denat. | 8 |
| Titandioxid | 0,5 |
| Ethylhexyl Methoxycinnamat | 9,5 |
| Butyl Methoxydibenzoylmethan | 4,5 |
| Phenylbenzimidazol Sulfonsäure | 2,0 |
| Bis-Ethylhexyloxyphenol Methoxyphenyltriazin | 3,0 |
| Vitamin E Acetat | 0,1 |
| Na₂H₂EDTA | 0,2 |
| Parfüm, Konservierungsmittel | q.s. |
| Farbstoffe, usw. | q.s. |
| Citronensäure, Natriumcitrat | q.s. |
| Natriumhydroxid | q.s. |
| Wasser | ad 100,0 |

### Muster 2

### handelsübliche Sonnenschutzlotion

### Messungen:

- visuelle Beurteilung des Schwitzens
- individuelles Empfinden per Fragebogen
   - Beurteilung der Testfragen (VOR Saunaaufenthalt)
   - Beurteilung der Testfragen (WÄHREND Saunaaufenthalt)
   - Ende Saunagang (15-30 min)
   - Beurteilung der Testfragen (NACH Saunaaufenthalt)
- Bestimmung der aufgetragenen Produktmenge (kein Unterschied, Daten nicht gezeigt)

### Ergebnisse:

- visuelle Beurteilung des Schwitzens
   1. Muster 1 (erfindungsgemäß): 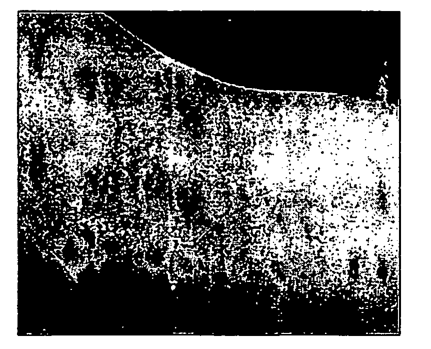
   2. Muster 2 (nicht erfindungsgemäße handelsübliche Sonnenschutzlotion): 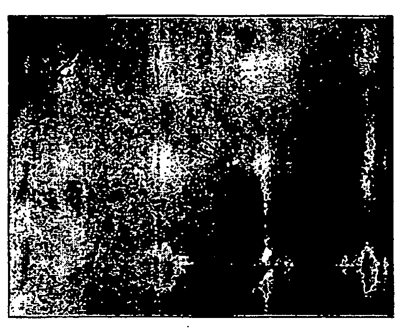
- individuelles Empfinden per Fragebogen

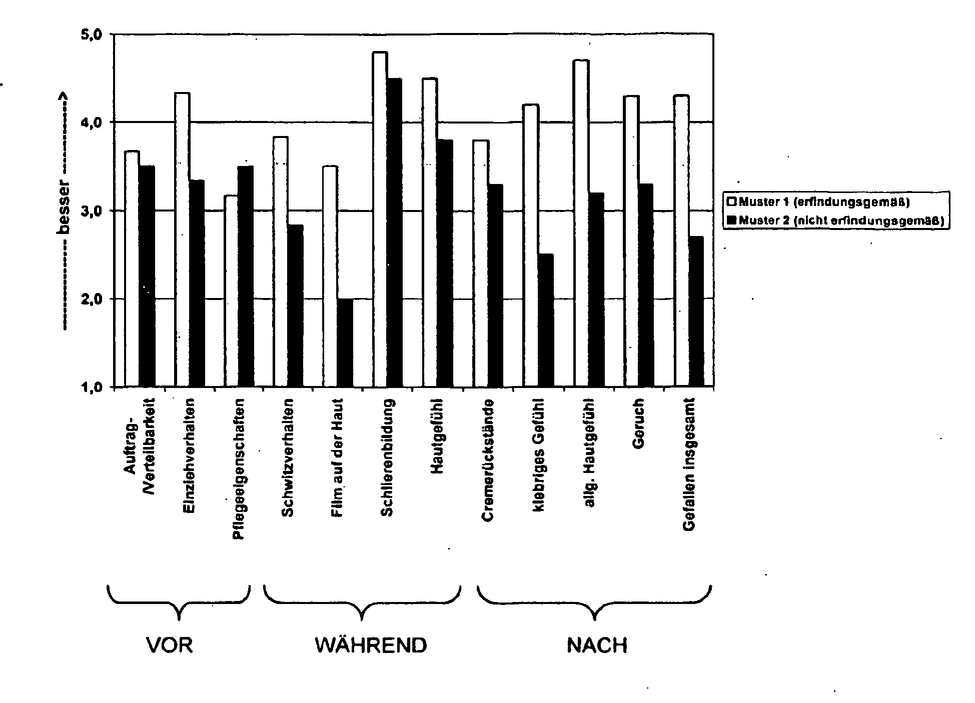

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

| | **1** | **2** | **3** | **4** |
|---|---|---|---|---|
| Glyceryl Stearat SE | 1 | 1 | 2 | 1 |
| Cetearyl Alkohol | 1,9 | 2,0 | 1,35 | 2,0 |
| PEG-40 Castoröl | 0,375 | 0,4 | 0,225 | 0,4 |
| Natrium Cetearyl Sulfat | 0,19 | 0,18 | 0,11 | 0,18 |
| Cetylalkohol | 2,2 | 2 | 1,8 | 2 |
| Myristylmyristat | 1,5 | 3 | 2 | 0,5 |
| Xanthan Gum | 0,2 | 0,3 | 0,4 | |
| C₁₂₋₁₅ Alkyl Benzoat | 6 | 5 | 7 | |
| 2-Phenylethylbenzoat | | 1 | | 2 |
| Caprylic/Capric Triglycerid | 1 | 2 | 2,5 | |
| Tridecylsalicylat | 1 | | | 1 |
| Distarch Phosphat | 3 | 2 | 2,5 | 2 |
| Natriumstärke Octenylsuccinat | 1,5 | 1 | 2 | |
| Cydomethicon | | | 2 | |
| Dimethicon | | 1 | | 2 |
| PVP Hexadecen Copolymer | | | 1 | |
| Glycerin | 0,9 | 1 | 1,5 | 3 |
| Alkohol denat. | 7 | 8 | 6 | 10 |
| Titandioxid | 0,75 | 0,25 | 0,5 | |
| Merocyanin | | 1 | | |
| Ethylhexyltriazin | 0,75 | 0,5 | 0,25 | 2 |
| Octyldodecanol | 2 | | | |
| Ethylhexyl Methoxycinnamat | 5 | 3 | 1 | 4,5 |
| Butyl Methoxydibenzoylmethan | 3,4 | 2,0 | 1 | 4,5 |
| Octocrylen | | | | 5 |
| Phenylbenzimidazol Sulfonsäure | 1,5 | 1 | 0,5 | |
| Methylpropandiol | | | 3 | |
| Ethylhexylsalicylat | | | 4,5 | |
| Bis-Ethylhexyloxyphenol Methoxyphenyltriazin | 1,7 | 1 | 2,5 | 3,5 |
| Diethylhexylbutamidotriazon | | | | 1 |
| Vitamin E Acetat | | 0,1 | 0,3 | 0,3 |
| Na₂H₂EDTA | 0,2 | 0,2 | 0,5 | 0,5 |
| Parfüm, Konservierungsmittel | q.s. | q.s. | q.s. | q.s. |
| Farbstoffe, usw. | q.s. | q.s. | q.s. | q.s. |
| Citronensäure, Natriumcitrat | q.s. | q.s. | q.s. | q.s. |
| Natriumhydroxid | q.s. | q.s. | q.s. | q.s. |
| Wasser | ad 100,0 | ad 100,0 | ad 100,0 | ad 100,0 |

| | 5 | 6 | 7 | 8 | 9 |
|---|---|---|---|---|---|
| Bis-Ethylhexyloxyphenol Methoxyphenyl Triazin | | 2,5 | 2 | 4 | 0,8 |
| Butyl Methoxydibenzoylmethan | 0,3 | 4,5 | 4 | 2,25 | 1,5 |
| Ethylhexyl Methoxycinnamat | 2 | 9,5 | 5,25 | 3,5 | 1,75 |
| Ethylhexyl Triazone | 0,3 | 1 | | 0,5 | |
| Phenylbenzimidazole Sulfonic Acid | | 3 | 1,5 | 1 | 0,5 |
| Titanium Dioxide + | | | | | |
| Trimethoxycaprylylsilane | | 1 | 2 | 0,5 | 0,5 |
| C12-15 Alkyl Benzoate | 8 | 6,5 | 6,5 | 6,5 | 6,5 |
| Caprylic/Capric Triglyceride | 3,5 | 2 | 1 | 5 | 3 |
| Cetearyl Alcohol | 2,5 | 3 | 2 | 5 | 20 |
| PEG-40 Castor Öl | | | 2 5 | | |
| Natrium Cetearyl Sulfat | 0,2 | 0,15 | 0,13 | 0,1 | 0,12 |
| Cetyl Alcohol | 1,5 | 2,2 | 2 | 1,5 | 2 |
| Alcohol Denat. | 8 | 6 | 5 | 3 | 10 |
| Parfum | 0,3 | 0,3 | 0,3 | 0,3 | 0,3 |
| Glycerin | 3 | 2 | 0,9 | 1 | 2 |
| Glyceryl Stearate SE | 1 | 0,65 | 0,65 | 0,65 | 0,65 |
| Methylparaben | | 0,3 | 0,3 | 0,3 | 0,3 |
| Myristyl Myristat | 1,5 | 1,5 | 1,5 | 1,5 | 1,5 |
| Octyldodecanol | 7,5 | 5,5 | 5,5 | 5,5 | 5,5 |
| Phenoxyethanol | 0,2 | | 0,2 | | 0,2 |
| Natrium Stärke Octenylsuccinat | 1,5 | 1,2 | 1,5 | 1 | 1,5 |
| Hydroxyethylurea | 2 | | 5 | | |
| Distärke Phosphat | 3,5 | 3 | 4 | 3 | 2,5 |
| Vitamin E Acetat | 0,1 | 0,1 | 0,1 | 0,1 | 0,1 |
| Na2H2EDTA | 1 | 1 | 1 | 1 | 1 |
| Natriumhydroxid | | 0,6 | 0,45 | 0,3 | 0,15 |
| Xanthan Gummi | 0,4 | 0,4 | 0,4 | 0,4 | 0,4 |
| Wasser | ad 100,0 | ad 100,0 | ad 100,0 | ad 100,0 | ad 100,0 |

## Patentansprüche

1. Kosmetische Emulsion enthaltend eine Kombination aus
a) 0,5 bis 3 Gew.-% Glycerylstearat SE
b) 1 bis 5 Gew.-% Cetearylalkohol,
c) 0,01 bis 0,5 Gew.-% PEG-40 Rizinusöl,
d) 0,05 bis 0,25 Gew.-% Natriumcetearylsulfat und
e) 0,5 bis 3 Gew.-% Myristylmyristat,
jeweils bezogen auf das Gesamtgewicht der Zubereitung.

2. Kosmetische Emulsion nach Anspruch 1, **dadurch gekennzeichnet, dass** die Zubereitung als weitere Bestandteile einen oder mehrere UV-Lichtschutzfilter enthält.

3. Kosmetische Zubereitung nach einem der vorhergehenden Ansprüche, **dadurch gekennzeichnet, dass** die Zubereitung einen oder mehrere UV-Lichtschutzfilter gewählt aus der Gruppe der Verbindungen Phenylen-1,4-bis-(2-benzimidazyl)-3,3'-5,5'-tetrasulfonsäuresalze; 2-Phenylbenzimidazol-5-sulfonsäuresalze; 1,4-di(2-oxo-10-Sulfo-3-bornylidenmethyl)-Benzol und dessen Salze; 4-(2-Oxo-3-bomylidenmethyl)benzolsulfonsäuresalze; 2-Methyl-5-(2-oxo-3-bornylidenmethyl)sulfonsäuresalze; 2,2'-Methylen-bis-(6-(2H-benzotriazol-2-yl)-4-(1,1,3,3-tetramethylbutyl)-phenol); 2-(2H-benzotriazo-2-yl)-4-methyl-6-[2-methyl-3-[1,3,3,3-tetramethyl-1-[(trimethylsilyl)oxy]disiloxanyl]propyl]-phenol; 3-(4-Methylbenzyliden)campher; 3-Benzylidencampher; Ethylhexylsalicylat; Terephthalidendicamphersulfonsäure; 4-(Dimethylamino)-benzoesäure(2-ethylhexyl)ester; 4-(Dimethylamino)benzoesäure-amylester; 4-Methoxybenzalmalon-säuredi(2-ethylhexyl)ester; 4-Methoxyzimtsäure(2-ethylhexyl)ester; 4-Methoxyzimtsäureisoamylester; 2-Hydroxy-4-methoxybenzophenon, 2-Hydroxy-4-methoxy-4'-methylbenzophenon; 2,2'-Dihydroxy-4-methoxybenzophenon; 2-(4'-Diethylamino-2'-hydoxybenzoyl)-benzoesäurehexylester, 4-(tert.-Butyl)-4'-methoxydibenzoylmethan; Homomenthylsalicylat; 2-Ethylhexyl-2-hydroxybenzoat; 2-Ethylhexyl-2-cyano-3,3-diphenylacrylat; Dimethicodiethylbenzalmalonat; 3-(4-(2,2-bis Ethoxycarbonylvinyl)-phenoxy)propenyl)-methoxysiloxan / Dimethylsiloxan - Copolymer; 2,4-Bis-{[4-(2-ethyl-hexyloxy)-2-hydroxy]-phenyl}-6-(4-methoxyphenyl)-1,3,5-triazin (INCI :Bis-Ethylhexyloxyphenol Methoxyphenyl Triazin); Dioctylbutylamidotriazon (INCI: Diethylhexyl-Butamidotriazone); 2,4-bis-[5-1(dimethylpropyl)benzoxazol-2-yl-(4-phenyl)-imino]-6-(2-ethylhexyl)-imino-1,3,5-triazin mit der (CAS Nr. 288254-16-0); 4,4',4"-(1,3,5-Triazin-2,4,6-triyltriimino)-tris-benzoësäure-tris(2-ethylhexylester) (auch: 2,4,6-Tris-[anilino-(p-carbo-2'-ethyl-1'-hexyloxy)]-1,3,5-triazin (INCI: Ethylhexyl Triazone); 2,4,6-Tribiphenyl-4-yl-1,3,5-triazin; Merocyanine; Benzoylzimtsäurenitrile, Titiandioxid; Zinkoxid in einer Konzentration von 0,01 bis 40 Gewichts-% bezogen auf das Gesamtgewicht der Zubereitung.

4. Kosmetische Zubereitung nach einem der vorhergehenden Ansprüche, **dadurch gekennzeichnet, dass** die Zubereitung ein oder mehrere Verbindungen gewählt aus der Gruppe der Parabene, Phenoxyethanol, Ethylhexylglycerin, 2-Methylpropan-1,3-diol, Butylenglycol, Propylenglycol enthält.

5. Kosmetische Zubereitung nach einem der vorhergehenden Ansprüche, **dadurch gekennzeichnet, dass** die Zubereitung als UV-Filter Titandioxid in einer Konzentration von 0,1 bis 5 Gew.-%, bezogen auf das Gesamtgewicht der Zubereitung enthält.

6. Kosmetische Zubereitung nach einem der vorhergehenden Ansprüche, **dadurch gekennzeichnet, dass** die Zubereitung bis zu 5 Gew.-% Glycerin, bezogen auf das Gesamtgewicht der Zubereitung, enthält.

7. Kosmetische Zubereitung nach einem der vorhergehenden Ansprüche, **dadurch gekennzeichnet, dass** die Zubereitung bis zu 15 Gew.-% Ethanol, bezogen auf das Gesamtgewicht der Zubereitung, enthält.

8. Kosmetische Zubereitung nach einem der vorhergehenden Ansprüche, **dadurch gekennzeichnet, dass** die Zubereitung bis zu 5 Gew.-% Stärkederivate, bezogen auf das Gesamtgewicht der Zubereitung, enthält.

9. Kosmetische Zubereitung nach einem der vorhergehenden Ansprüche, **dadurch gekennzeichnet, dass** die Stärkederivate gewählt werden aus der Gruppe der Verbindungen Distärkephosphat, Natriumoctenylsuccinatstärke.

10. Kosmetische Zubereitung nach einem der vorhergehenden Ansprüche, **dadurch gekennzeichnet, dass** ein oder mehrere Ölkomponenten gewählt aus der Gruppe der Verbindungen C₁₂₋₁₅-Alkylbenzoat, Capryl-/Caprinsäure Triglycerid, Dicaprylcarbonat.

## Claims

1. Cosmetic emulsion comprising a combination of
a) 0.5 to 3% by weight of glyceryl stearate SE
b) 1 to 5% by weight of cetearyl alcohol,
c) 0.01 to 0.5% by weight of PEG-40 castor oil,
d) 0.05 to 0.25% by weight of sodium cetearyl sulphate and
e) 0.5 to 3% by weight of myristyl myristate,
based in each case on the total weight of the formulation.

2. Cosmetic emulsion according to Claim 1, **characterized in that** the formulation comprises, as further constituents, one or more photoprotective UV filters.

3. Cosmetic formulation according to either of the preceding claims, **characterized in that** the formulation comprises one or more photoprotective UV filters selected from the group of the compounds phenylene-1,4-bis(2-benzimidazyl)-3,3',5,5'-tetrasulphonic acid salts, 2-phenylbenzimidazole-5-sulphonic acid salts; 1,4-di-(2-oxo-10-sulpho-3-bornylidenemethyl)benzene and salts thereof, 4-(2-oxo-3-bornylidenemethyl)-benzenesulphonic acid salts, 2-methyl-5-(2-oxo-3-bornylidenemethyl)sulphonic acid salts; 2,2'-methylenebis (6-(2H-benzotriazol-2-yl)-4-(1,1,3,3-tetramethylbutyl)phenol; 2-(2H-benzotriazol-2-yl)-4-methyl-6-[2-methyl-3-[1,3,3,3-tetramethyl-1-[(trimethylsilyl)oxy]disiloxanyl]propyl]phenol; 3-(4-methylbenzylidene)camphor; 3-benzylidenecamphor; ethylhexyl salicylate; terephthalidene-dicamphorsulphonic acid; 4-(dimethylamino)benzoic acid 2-ethylhexyl ester; 4-(dimethylamino)benzoic acid amyl ester; 4-methoxybenzalmalonic acid di(2-ethylhexyl) ester; 4-methoxycinnamic acid 2-ethylhexyl ester; 4-methoxycinnamic acid isoamyl ester; 2-hydroxy-4-methoxybenzophenone, 2-hydroxy-4-methoxy-4'-methylbenzophenone; 2,2'-dihydroxy-4-methoxybenzophenone; 2-(4'-diethylamino-2'-hydroxybenzoyl)benzoic acid hexyl ester, 4-(tert-butyl)-4'-methoxydibenzoylmethane; homomenthyl salicylate; 2-ethylhexyl 2-hydroxybenzoate; 2-ethylhexyl 2-cyano-3,3-diphenylacrylate; dimethicodiethyl benzalmalonate; 3-(4-(2,2-bis ethoxycarbonylvinyl)phenoxy)propenyl)methoxy-siloxane/dimethylsiloxane copolymer; 2,4-bis{[4-(2-ethylhexyloxy)-2-hydroxy]phenyl}-6-(4-methoxyphenyl)-1,3,5-triazine (INCI: Bis-Ethylhexyloxyphenol Methoxyphenyl Triazine); dioctylbutylamidotriazone (INCI: Diethylhexyl-Butamidotriazone); 2,4-bis[5-(dimethylpropyl)benzoxazol-2-yl-(4-phenyl)imino]-6-(2-ethylhexyl)imino-1,3,5-triazine with CAS No. 288254-16-0; 4,4',4''-(1,3,5-triazine-2,4,6-triyltriimino)trisbenzoic acid tris(2-ethylhexyl) ester (also: 2,4,6-tris-[anilino-(p-carbo-2'-ethyl-1'-hexyloxy)]-1,3,5-triazine (INCI: Ethylhexyl Triazone)); 2,4,6-tribiphenyl-4-yl-1,3,5-triazine; merocyanines; benzoylcinnamonitriles, titanium dioxide; zinc oxide in a concentration of 0.01 to 40% by weight, based on the total weight of the formulation.

4. Cosmetic formulation according to any of the preceding claims, **characterized in that** the formulation comprises one or more compounds selected from the group of the parabens, phenoxyethanol, ethylhexylglycerol, 2-methylpropane-1,3-diol, butylene glycol, propylene glycol.

5. Cosmetic formulation according to any of the preceding claims, **characterized in that** the formulation comprises, as a UV filter, titanium dioxide in a concentration of 0.1 to 5% by weight, based on the total weight of the formulation.

6. Cosmetic formulation according to any of the preceding claims, **characterized in that** the formulation comprises up to 5% by weight of glycerol, based on the total weight of the formulation.

7. Cosmetic formulation according to any of the preceding claims, **characterized in that** the formulation comprises up to 15% by weight of ethanol, based on the total weight of the formulation.

8. Cosmetic formulation according to any of the preceding claims, **characterized in that** the formulation comprises up to 5% by weight of starch derivatives, based on the total weight of the formulation.

9. Cosmetic formulation according to any of the preceding claims, **characterized in that** the starch derivatives are selected from the group of the compounds distarch phosphate, sodium octenyl-succinate starch.

10. Cosmetic formulation according to any of the preceding claims, **characterized in that** one or more oil components are selected from the group of the compounds C₁₂₋₁₅-alkyl benzoate, caprylic capric triglyceride, dicapryl carbonate.

## Revendications

1. Émulsion cosmétique contenant une association de
a) 0,5 à 3 % en poids de stéarate de glycéryle SE
b) 1 à 5 % en poids d'alcool cétéarylique,
c) 0,01 à 0,5 % en poids de PEG-40 huile de ricin,
d) 0,05 à 0,25 % en poids de cétéarylsulfate de sodium et
e) 0,5 à 3 % en poids de myristate de myristyle,
chaque fois par rapport au poids total de la préparation.

2. Émulsion cosmétique selon la revendication 1, **caractérisée en ce que** la préparation contient comme autres composants un ou plusieurs filtres photoprotecteurs UV.

3. Préparation cosmétique selon l'une quelconque des revendications précédentes, **caractérisée en ce que** la préparation contient un ou plusieurs filtres photoprotecteurs UV choisis dans le groupe des composés sels d'acide phénylène-1,4-bis-(2-benzimidazyl)-3,3'-5,5'-tétrasulfonique ; sels d'acide 2-phényl-benzimidazole-5-sulfonique ; 1,4-di(2-oxo-10-sulfo-3-bornylidène-méthyl)-benzène et ses sels ; sels d'acide 4-(2-oxo-3-bornylidéne-méthyl)benzénesulfonique ; sels d'acide 2-méthyl-5-(2-oxo-3-bornylidène-méthyl)-sulfonique ; 2,2'-méthyléne-bis(6-(2H-benzotriazol-2-yl)-4-(1,1,3,3-tétraméthylbutyl)-phénol) ; 2-(2H-benzo-triazol-2-yl)-4-méthyl-6-[2-méthyl-3-[1,3,3,3-tétraméthyl-1-[(triméthylsilyl)oxy]disiloxanyl]propyl]-phénol ; 3-(4-méthylbenzylidène)camphre ; 3-benzylidènecamphre ; salicylate d'éthylhexyle ; acide téréphtalidène-dicamphosulfonique ; 4-(diméthylamino)benzoate de 2-éthylhexyle ; 4-(diméthylamino)benzoate d'amyle ; 4-méthoxybenzal-malonate de di(2-éthylhexyle) ; 4-méthoxycinnamate de 2-éthylhexyle ; 4-méthoxycinnamate d'isoamyle ; 2-hydroxy-4-méthoxybenzophénone, 2-hydroxy-4-méthoxy-4'-méthylbenzophénone ; 2,2'-dihydroxy-4-méthoxy-benzophénone ; 2-(4'-diéthylamino-2'-hydoxybenzoyl)-benzoate d'hexyle, 4-(tert-butyl)-4'-méthoxy-dibenzoylméthane : salicylate d'homomenthyle ; 2-hydroxybenzoate de 2-éthylhexyle ; 2-cyano-3,3-diphénylacrylate de 2-éthylhexyle ; benzalmalonate de diméthicodiéthyle ; copolymère 3-(4-(2,2-bis-éthoxycarbonylvinyl)-phénoxy)propényl)-méthoxysiloxane / diméthylsiloxane ; 2,4-bis-{[4-(2-éthyl-hexyloxy)-2-hydroxy]-phényl}-6-(4-méthoxyphényl)-1,3,5-triazine (INCI : Bis-Ethylhexyloxyphénol Methoxyphenyl Triazin) ; dioctylbutylamidotriazone (INCI : Diethylhexyl-Butamidotriazone) ; 2,4-bis-[5-1(diméthylpropyl)benzoxazol-2-yl-(4-phényl)imino]-6-(2-éthylhexyl)-imino-1,3,5-triazine portant le (n° CAS 288254-16-0) ; 4,4',4"-(1,3,5-triazine-2,4,6-triyltriimino)-tris-benzoate de tris(2-éthylhexyle) (également : 2,4,6-tris[anilino-(p-carbo-2'-éthyl-1'-hexyloxy)]-1,3,5-triazine (INCI : Ethylhexyl Triazone) ; 2,4,6-tribiphényl-4-yl-1,3,5-triazine ; mérocyanines ; benzoylcinnamonitriles, dioxyde de titane ; oxyde de zinc à une concentration de 0,01 à 40 % en poids par rapport au poids total de la préparation.

4. Préparation cosmétique selon l'une quelconque des revendications précédentes, **caractérisée en ce que** la préparation contient un ou plusieurs composés choisis dans le groupe constitué par les parabens, le phénoxyéthanol, l'éthylhexylglycérol, le 2-méthyl-propane-1,3-diol, le butylèneglycol, le propylène-glycol.

5. Préparation cosmétique selon l'une quelconque des revendications précédentes, **caractérisée en ce que** la préparation contient en tant que filtre UV du dioxyde de titane à une concentration de 0,1 à 5 % en poids par rapport au poids total de la préparation.

6. Préparation cosmétique selon l'une quelconque des revendications précédentes, **caractérisée en ce que** la préparation contient jusqu'à 5 % en poids de glycérol, par rapport au poids total de la préparation.

7. Préparation cosmétique selon l'une quelconque des revendications précédentes, **caractérisée en ce que** la préparation contient jusqu'à 15 % en poids d'éthanol, par rapport au poids total de la préparation.

8. Préparation cosmétique selon l'une quelconque des revendications précédentes, **caractérisée en ce que** la préparation contient jusqu'à 5 % en poids de dérivés d'amidon, par rapport au poids total de la préparation.

9. Préparation cosmétique selon l'une quelconque des revendications précédentes, **caractérisée en ce que** les dérivés d'amidon sont choisis dans le groupe des composés phosphate de diamidon, octénylsuccinate sodique-amidon.

10. Préparation cosmétique selon l'une quelconque des revendications précédentes, **caractérisée en ce qu'**un ou plusieurs composants huileux sont choisis dans le groupe des composés benzoate d'alkyle en C₁₂-C₁₅, triglycéride d'acides caprique/caprylique, carbonate de dicapryle.
